**Europäisches Patentamt**

(19) **European Patent Office**

**Office européen des brevets**

(11) Publication number: **0 122 025**
**B1**

(12) **EUROPEAN PATENT SPECIFICATION**

(45) Date of publication of patent specification: **23.09.87**

(21) Application number: **84301517.3**

(22) Date of filing: **07.03.84**

(51) Int. Cl.⁴: **C 07 D 301/02, B 01 J 23/04**

(54) Method for the manufacture of epoxides from 1,2-glycols.

(30) Priority: **09.03.83 US 473838**

(43) Date of publication of application:
**17.10.84 Bulletin 84/42**

(45) Publication of the grant of the patent:
**23.09.87 Bulletin 87/39**

(84) Designated Contracting States:
**BE DE FR GB IT NL**

(56) References cited:
**EP-A-0 002 561**
**DE-A-1 940 205**
**FR-A-2 345 440**
**FR-A-2 382 446**

(73) Proprietor: **ATLANTIC RICHFIELD COMPANY**
**515 South Flower Street**
**Los Angeles California 90071 (US)**

(72) Inventor: **Gastinger, Robert G.**
**4502 School Lane**
**Brookhaven, PA 19015 (US)**

(74) Representative: **Cropp, John Anthony David et al**
**MATHYS & SQUIRE 10 Fleet Street**
**London, EC4Y 1AY (GB)**

The file contains technical information submitted after the application was filed and not included in this specification

# Description

This invention relates to the manufacture of epoxides.

More specifically, this invention relates to the manufacture of epoxides from 1,2 glycols.

In one of its more specific aspects, this invention pertains to a method for converting 1,2 propylene glycol directly to propylene oxide without forming an ester intermediate.

The vapor phase and liquid phase cracking of propylene glycol monoacetate ester to propylene oxide is well known. In general for this process feeds include various mixtures of propylene glycol mono- and di-acetate often containing some propylene glycol. However, an available source of ester, usually from the acetoxylation of propylene, is required or is generated in situ through, for example, the use of an alkali metal acetate supported catalyst. U.S. 4,226,780 teaches the manufacture of propylene oxide from 1,2 propylene glycol using as the catalyst a weakly acidic carrier and an added basic alkali metal salt of a low molecular weight carboxylic acid.

The vapor phase dehydration of 1,2 glycols to epoxides has also been proposed. FR—A—2345440 describes obtaining epoxides from alkane-1,2-diols by contacting the latter in the vapor phase and at a temperature of 250 to 550°C with a basic substance. Selectivities in the region of about 30% to about 50% are reported in Examples using alkali metal compounds as catalysts.

We have now found that high epoxide yields are obtainable with substantially higher selectivities if potassium silicate is employed as catalyst.

According to this invention there is provided a method for the production of epoxides which comprises heating a 1,2 glycol in the vapor phase at a reaction temperature of from about 250 to 500°C over a catalyst which consists essentially of potassium silicate supported or unsupported on a carrier.

In a preferred embodiment, the process of this invention is employed to produce propylene oxide directly from 1,2 propylene glycol.

In another embodiment, it has been found that the addition of acetic acid to the glycol feed significantly enhances epoxide selectivity without affecting catalyst activity.

Carriers found useful are α-alumina, zircon and a refractory metal oxide consisting of 94% by weigh α-alumina, 5% by weight CaO, and 1% CuO. In general for supported silicates 5—20 weight percent of the salt on the carrier is employed.

In the practice of this invention, 1,2-glycol feed concentrations, acetic acid feed concentrations and residence times based on catalyst void space can be varied between wide ranges. Preferably, 1,2-glycol feed concentrations will be within the range of 10—70 mol %. Acetic acid feed concentration, if employed, will be within the range of from about 0.5 to 5 mol % and residence times will be within the range of from about 0.5 to 2 seconds.

The following examples further demonstrate catalyst preparation and the manufacture of epoxides according to this invention.

## Example I

The example demonstrates the preparation of a potassium silicate catalyst and its use to produce propylene oxide from propylene glycol. Acetic acid is employed in the feed stream to improve propylene oxide selectivity.

About 18.6 grams of potassium silicate were calcined at 450°C in air for about 12 hours and sieved to 8—14 mesh.

A stainless steel tubular reactor about 14.3 mm (9/16'') internal diameter (ID) was packed with 30 ml of the potassium silicate catalyst.

At 398°C a feed stream of 38 mol % propylene glycol and 1.5 mol % acetic acid in nitrogen was vaporized and passed over the catalyst at a rate of 26.3 g propylene glycol per hour.

The products were collected and analyzed by gas chromatography. The conversion of propylene glycol was 21% with a 79% propylene oxide and 11% propanal selectivity. The remaining products consisted primarily of acetone, allyl alcohol, 1-propanol, propylene and acetol (hyroxyacetone).

## Example II

This example demonstrates the preparation of a potassium silicate catalyst supported on a refractory metal oxide and its use to produce propylene oxide from propylene glycol. Acetic acid is employed in the feed stream to improve propylene oxide selectivity.

A concentrated aqueous solution of 3.23 g potassium silicate was impregnated on 42.6 g of refractory metal oxide (containing 94% $AL_2O_3$). The resulting material was air calcined at 470°C for 10 hours, pelletized and sieved to 8—14 mesh.

A stainless steel tubular reactor about 14.3 mm (9/16'') ID was packed with 25 ml of the catalyst.

At 399°C a feed stream of 37 mol % propylene glycol and 1.5 mol % acetic acid in nitrogen was vaporized and passed over the catalyst at a rate of 18.5 g propylene glycol per hour.

The products were collected and analyzed by gas chromatography. The conversion of propylene glycol was 26% with a 77% propylene oxide and 14% propanal selectivity. The remaining products consisted primarily of acetone, allyl alcohol, 1-propanol, propylene and acetol (hyroxyacetone).

## Example III

This example demonstrates the preparation of a potassium silicate catalyst supported in α-alumina and its use to produce propylene oxide from propylene glycol.

A solution of 5.00 g potassium silicate in 18 ml of water was added to 50.0 g of α-alumina. The resulting impregnated alumina was then dried and air calcined at 425°C for 10 hours, pelletized and sieved to 8—14 mesh.

A stainless steel tubular reactor about 14.3 mm (9/16'') ID was packed with 30 ml of the potassium silicate catalyst.

At 400°C a feed stream of 34 mol % propylene glycol in nitrogen was vaporized and passed over the catalyst at a rate of 25.6 g propylene glycol per hour.

The products were collected and analyzed by gas chromatography. The conversion of propylene glycol was 39% with a 82% propylene oxide and 7% propanal selectivity. The remaining products consisted primarily of acetone, allyl alcohol, 1-propanol, propylene and acetol (hyroxyacetone).

### Example IV

This example demonstrates the preparation of a potassium silicate catalyst supported on zircon and its use to produce propylene oxide from propylene glycol.

A solution of 8.50 g potassium silicate in 30 ml of water was added to 42.6 zirconium silicate. The water was removed in vacuo with heating and the resulting material was air calcined at 450°C for 10 hours, pelletized and sieved to 8—14 mesh.

A stainless steel tubular reactor about 14.3 mm (9/16'') ID was packed with 25 ml of the potassium silicate catalyst.

At 450°C, a feed stream of 30 mol % propylene glycol in nitrogen was vaporized and passed over the catalyst at a rate of 18.8 g propylene glycol per hour.

The products were collected and analyzed by gas chromatography. The conversion of propylene glycol was 59% with a 74% propylene oxide and 13% propanal selectivity. The remaining products consisted primarily of acetone, allyl alcohol, 1-propanol, propylene and acetol (hyroxyacetone).

## Claims

1. A method for the preparation of an epoxide by contacting a 1,2 glycol stream in the vapor phase and at a temperature of from 250 to 500°C with an alkali metal compound as catalyst characterised in that the catalyst consists essentially of potassium silicate supported or unsupported on a carrier.

2. A method as claimed in claim 1 characterised in that an effective amount of acetic acid is added to the 1,2 glycol stream to improve selectivity.

3. A method as claimed in claim 1 or claim 2 in which propylene oxide is obtained from a 1,2 propylene glycol stream.

## Patentansprüche

1. Verfahren zur Herstellung eines Epoxids durch Inkontaktbringen eines 1,2-Glykolstroms in der Dampfphase und bei einer Temperatur von 250 bis 500°C mit einer Alkalimetallverbindung als Katalysator, dadurch gekennzeichnet, daß der Katalysator im wesentlichen Kaliumsilikat gestützt oder ungestützt auf einem Träger enthält.

2. Verfahren nach Anspruch 1, dadurch gekennzeichnet, daß man zur Erhöhung der Selektivität eine wirksame Menge Essigsäure zum 1,2-Glykolstrom gibt.

3. Verfahren nach Anspruch 1 oder 2, worin man aus einem 1,2-Propylenglykolstrom Propylenoxid erhält.

## Revendications

1. Procédé de préparation d'un époxyde par contact d'un courant de 1,2-glycol en phase gazeuse et à une température de 250 à 500°C avec un composé de métal alcalin en tant que catalyseur, caractérisé en ce que le catalyseur consiste essentiellement en silicate de potassium supporté ou non-supporté sur un support.

2. Procédé suivant la revendication 1, caractérisé en ce qu'une quantité efficace d'acide acétique est ajoutée au courant de 1,2-glycol pour améliorer la sélectivité.

3. Procédé suivant la revendication 1 ou la revendication 2, dans lequel l'oxyde de propylène est obtenu à partir d'un courant de 1,2-propylène glycol.